# EUROPÄISCHE PATENTSCHRIFT

(11) **EP 0 437 764 B1**
(45) Veröffentlichungstag und Bekanntmachung des Hinweises auf die Patenterteilung: **28.07.1993**
(21) Anmeldenummer: 90124486.3
(22) Anmeldetag: 18.12.1990
(51) Int. Cl.: C07C 45/90, C07C 49/88, D21H 17/17

(54) **Stabilisierte wässrige Alkyldiketenemulsionen**
Stable aqueous emulsions of alkyl-keten dimer
Emulsions aqueuses stables de dimère cétène-alcyle

(30) Priorität: 18.01.1990 DE 4001237
(43) Veröffentlichungstag der Anmeldung: 24.07.1991
(73) Patentinhaber: BASF Aktiengesellschaft, 67063 Ludwigshafen (DE)
(72) Erfinder: Ksoll, Peter, Dr., W-6915 Dossenheim (DE); Hahn, Erwin, Dr., W-6900 Heidelberg (DE); Wittmer, Peter, W-6730 Neustadt (DE); Hohmann, Andreas, Dr., W-6700 Ludwigshafen (DE); De Clerq, Arnold, Dr., W-6716 Dirmstein (DE); Riebeling, Ulrich, W-6707 Schifferstadt (DE)

(56) Entgegenhaltungen:
- EP-A- 0 093 321
- EP-A- 0 327 215
- US-A- 2 627 477
- US-A- 2 901 371
- US-A- 3 311 532
- US-A-31 301 18
- US-H-93 600 3

## Beschreibung

Die Erfindung betrifft stabilisierte wäßrige Alkyldiketenemulsionen, die mindestens 10 Gew.-% eines C₁₄- bis C₂₂-Alkyldiketens emulgiert enthalten und die Verwendung von langkettigen Fettsäureestern als Stabilisator zur Herstellung der Alkyldiketenemulsionen.

Aus der US-PS 2 627 477 ist bekannt, daß man Alkyldiketene mit mindestens 6 Kohlenstoffatomen im Molekül unter Zuhilfenahme von Emulgatoren oder organischen Verdickungsmitteln in Wasser emulgieren kann. Die dabei erhältlichen Diketenemulsionen sind Masseleimungsmittel für Papier. Aus der US-PS 3 130 118 ist bekannt, daß man Alkyldiketene mit mindestens 6 Kohlenstoffatomen in Gegenwart von kationischer Stärke in Wasser emulgieren kann. Die so erhältlichen niedrig konzentrierten Alkyldiketenemulsionen sind für eine Anwendung ausreichend lagerstabil, während wäßrige Emulsionen mit Konzentrationen an Fettalkyldiketen von mehr als 12 % relativ rasch fest werden.

Zur Stabilisierung von wäßrigen Fettalkyldiketenemulsionen, die durch Verwendung von kationischer Stärke hergestellt wurden, hat man bereits verschiedene Stoffe verwendet. So ist beispielsweise aus der US-PS 2 901 371 und der US-PS 3 311 532 bekannt, höhere Fettsäuren, ihre Anhydride, Amide, Aldehyde und Säurechloride zu verwenden. Die Konzentrationen an Fettalkyldiketen in den wäßrigen Emulsionen liegt dabei unter 10 Gew.-%.

Aus der EP-A-0 327 215 sind wäßrige Emulsionen bekannt, die ein Ketendimer, eine nicht reaktive hydrophobe Verbindung und einen Stabilisator enthalten. Die Feststoffkonzentration der Emulsionen beträgt 5 bis etwa 70 Gew.-%, das Gewichtsverhältnis von Ketendimer zu nicht reaktiver hydrophober Verbindung etwa 1:4 bis 1:166. Die Emulsionen werden zum Leimen von Zellulosefasern verwendet. Die Konzentrationen an Alkylketendimer in den wäßrigen Emulsionen beträgt gemäß den Beispielen nicht mehr als 6 Gew.-%.

Der vorliegenden Erfindung liegt die Aufgabe zugrunde, wäßrige Alkyldiketenemulsionen zur Verfügung zu stellen, die eine höhere Konzentration an Fettalkyldiketen aufweisen als die bekannten Alkyldiketenemulsionen und die dabei lagerstabil sind.

Die Aufgabe wird erfindungsgemäß gelöst mit stabilisierten wäßrigen C₁₄-bis C₂₂-Alkyldiketenemulsionen, wenn sie als wesentliche Bestandteile
(a) 10 bis 60 Gew.-% mindestens eines C₁₄- bis C₂₂-Alkyldiketens,
(b) 1 bis 10 Gew.-% mindestens eines Schutzkolloids und
(c) 0,1 bis 20 Gew.-% mindestens eines Esters der Formel

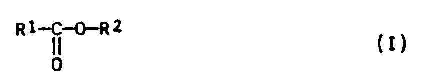

enthalten, in der R¹ und R²
- (1): C₁₄- bis C₂₂-Alkyl bedeuten, wobei sich R¹ und R² um mindestens 4 C-Atome in der Alkylkette unterscheiden,
- (2): R¹ = C₁₄- bis C₂₂-Alkyl
R² = C₁₄- bis C₂₂-Alkenyl
- (3): R¹ = C₁₄- bis C₂₂-Alkenyl
R² = C₁₄- bis C₂₂-Alkyl oder
- (4): R¹ und R² gleiche oder verschiedene C₁₄- bis C₂₂-Alkenyl bedeuten,

eines Esters der Formel 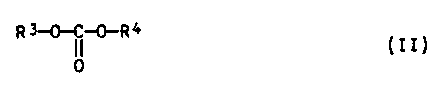
in der R³ und R⁴ gleiche oder verschiedene Alkyl- oder Alkenylreste mit 2 bis 22 C-Atomen bedeuten und R³ oder R⁴ mindestens 6 C-Atome aufweist, oder
eines Urethans der Formel 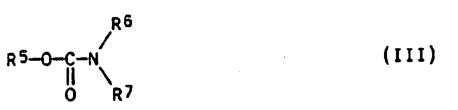
enthalten, in der R⁵, R⁶ und R⁷ gleiche oder verschiedene Alkyl- oder Alkenylreste mit 2 bis 22 C-Atome bedeuten und einer der Substituenten R⁵, R⁶ oder R⁷ mindestens 12 C-Atome aufweist,
und wenn das Verhältnis der emulgierten Bestandteile (a):(c) 200:1 bis 1:1 beträgt.

Die Verbindungen der Formeln I bis III sind überraschenderweise wirksame Stabilisatoren für wäßrige Alkyldiketenemulsionen, die hohe Feststoffgehalte aufweisen, z.B. mindestens 10 und vorzugsweise mindestens 16 Gew.-% Alkyldiketen. Sie werden daher auch als Stabilisatoren zur Herstellung von wäßrigen C₁₄- bis C₂₂-Alkyldiketenemulsionen, die mindestens 10 Gew.-% Alkyldiketen emulgiert enthalten, verwendet. Die Einsatzmengen an Stabilisator betragen dabei 0,5 bis 50 Gew.- %, bezogen auf Alkyldiketen.

Die Komponente (a) der stabilisierten wäßrigen Alkyldiketenemulsionen besteht aus mindestens einem C₁₄- bis C₂₂-Alkyldiketen oder einer Mischung aus solchen Alkyldiketenen. Die Alkyldiketene sind bekannt und im Handel erhältlich. Sie werden beispielsweise aus den entsprechenden Carbonsäurechloriden durch Abspaltung von Chlorwasserstoff mit tertiären Aminen hergestellt. Die Fettalkyldiketene können beispielsweise mit Hilfe der Formel II charakterisiert werden: 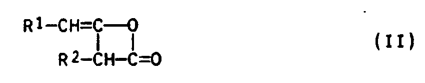
in der R¹, R² gleiche oder verschiedene C₁₄- bis C₂₂-Alkylgruppen bedeuten.

Geeignete Fettalkyldiketene sind beispielsweise Tetradecyldiketen, Hexadecyldiketen, Octadecyldiketen, Eikosyldiketen, Dokosyldiketen, Palmityldiketen, Stearyldiketen und Behenyldiketen. Beispiele für Verbindungen der Formel II, in der die Substituenten R¹ und R² eine unterschiedliche Bedeutung haben, sind beispielsweise Stearylpalmityldiketen, Behenylstearyldiketen, Behenyloleyldiketen oder Palmitylbehenyldiketen. Von den Verbindungen der Formel II verwendet man vorzugsweise Stearyldiketen, Palmityldiketen oder Mischungen aus Stearyldiketen und Palmityldiketen. Die Diketene sind in Konzentrationen von 10 bis 60, vorzugsweise 16 bis 40 Gew.-% in den wäßrigen Emulsionen enthalten. Besonders bevorzugt sind solche wäßrigen Alkyldiketenemulsionen, die Konzentrationen in dem Bereich von 20 bis 35 Gew.-% eines C₁₄- bis C₂₂-Alkyldiketens aufweisen.

Die Alkyldiketene werden üblicherweise in Gegenwart eines Schutzkolloids in Wasser emulgiert. Geeignete Schutzkolloide sind bereits in der grundlegenden US-PS 2 627 477 offenbart, und zwar beispielsweise Sorbitanester, wie Sorbitanmonopalmitat, polyalkoxylierte Derivate von Sorbitanestern, anionische oder nichtionische Emulgiermittel, wie verschiedene Seifen, synthetische Detergentien und Verdickungsmittel, wie Stärke und wasserlösliche Cellulosederivate. Weitere geeignete Schutzkolloide zur Herstellung der wäßrigen Alkyldiketenemulsionen sind beispielsweise Polymere von Vinylalkohol, Acrylamid, Vinylpyrrolidon oder N-Vinyl-2-methylimidazolin. Besonders bevorzugt ist die Verwendung von kationischen Stärken als Schutzkolloid. Geeignete kationische Stärken zum Emulgieren von Fettalkyldiketenen sind in der oben angegebenen US-PS-3 130 118 beschrieben und im Handel erhältlich. Die Menge an Schutzkolloid, vorzugsweise an kationischer Stärke, beträgt 1 bis 10, vorzugsweise 1,5 bis 3 Gew.-%, bezogen auf die gesamte Emulsion.

Zur Stabilisierung der wäßrigen C₁₄- bis C₂₂-Alkyldiketenemulsionen, die ein Schutzkolloid enthalten, verwendet man erfindungsgemäß mindestens einen Ester der Formel 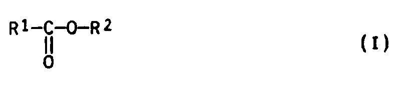
in der R¹ und R²
- (1): C₁₄- bis C₂₂-Alkyl bedeuten, wobei sich R¹ und R² um mindestens 4 C-Atome in der Alkylkette unterscheiden,
- (2): R¹ = C₁₄- bis C₂₂-Alkyl
R² = C₁₄- bis C₂₂-Alkenyl
- (3): R¹ = C₁₄- bis C₂₂-Alkenyl
R² = C₁₄- bis C₂₂-Alkyl oder
- (4): R¹ und R² gleiche oder verschiedene C₁₄- bis C₂₂-Alkenyl bedeuten.

Die Verbindungen der Formel I sind bekannt. Beispiele für geeignete Verbindungen der Formel I mit der oben unter (1) angegebenen Bedeutung für R¹ und R² sind Stearinsäurebehenylester, Behensäurestearylester, Myristinsäurestearylester, Myristinsäurebehenylester, Palmitinsäurebehenylester und Stearinsäureisododecylester.

Beispiele für die unter (2) angegebenen Bedeutungen für R¹ und R² sind Stearinsäureoleylester, Palmitinsäureoleylester, Behensäureoleylester, Myristinsäureoleylester und Arachinsäureoleylester.

Beispiele für die oben unter (3) angegebene Bedeutung der Substituenten R¹ und R² in Formel I sind Ölsäurestearylester, Ölsäurebehenylester und Ölsäurepalmitylester als Beispiel zu nennen. Geeignete Verbindungen mit der oben unter (4) angegebenen Bedeutung von R¹ und R² in Formel I sind Ölsäureoleylester, Ricinolsäureoleylester, Linolsäureoleylester und Tallölsäureoleylester. Besonders bevorzugt werden von den Verbindungen der Formel I als Komponente (c) der wäßrigen Alkyldiketenemulsionen Stearinsäureoleylester und Ölsäurestearylester eingesetzt.

Die Verbindungen der Formel R³-O-CO-O-R⁴ (II) sind ebenfalls bekannte Stoffe. Die Substituenten R³ und R⁴ bedeuten entweder gleiche oder verschiedene Alkyl- oder Alkenylreste, wobei mindestens einer der Substituenten R³ und R⁴ mindestens 6 C-Atome aufweist. Diese Substituenten können 2 bis 22 C-Atome enthalten. Sofern die Substituenten R³ und R⁴ Alkenyl bedeuten, enthält die Alkenylgruppe vorzugsweise mindestens 6 C-Atome. Beispiele für Verbindungen der Formel II sind Kohlensäureoleylstearylester, Kohlensäurebehenyloleylester, Kohlensäureethyloleylester, Kohlensäuredioleylester, Kohlensäurebehenylstearylester und Kohlensäure-(2-hexyl-decanyl)-oleylester.

Auch die Verbindungen der Formel 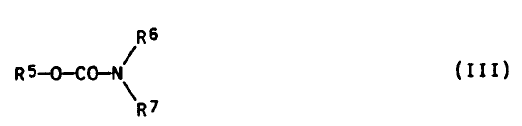
sind bekannte Stoffe. Die Substituenten R⁵, R⁶ und R⁷ sind entweder gleich oder verschieden. Sie können 2 bis 22 C-Atome aufweisen und stehen für eine Alkyl- oder Alkenylgruppe, wobei mindestens einer der Substituenten R⁵, R⁶ und R⁷ mindestens 12 C-Atome enthält. Sofern diese Substituenten für Alkenylgruppen stehen, beträgt die Anzahl der C-Atome der Alkenylgruppen im allgemeinen mindestens 12. Beispiele für Verbindungen der Formel III sind Oleyl-N,N-distearylurethan, Palmityl-N,N-distearylurethan, Oleyl-N-palmityl-N-stearylurethan und Behenyl-N,N-distearylurethan.

Die Verbindungen der Gruppe (c) sind in den wäßrigen Alkyldiketenemulsionen in Konzentrationen von 0,1 bis 20, vorzugsweise 1 bis 10 Gew.-% enthalten. Die Stabilisierung von insbesondere C₁₆- bis C₂₀-Alkyldiketenemulsionen einer Konzentration in dem Bereich von 20 bis 35 Gew.-% erfolgt im allgemeinen mit Mengen von 2 bis 8 Gew.-%, bezogen auf die Emulsion, einer Verbindung der Gruppe (c).

In der stabilisierten Dispersion liegen die oben unter (a) und (c) angegebenen Verbindungen in einem Verhältnis von 200:1 bis 1:1, vorzugsweise 20:1 bis 2:1 vor. Die Emulsionen werden in bekannter Weise in den dafür gebräuchlichen Apparaturen durch Emulgieren der hydrophoben Bestandteile in einer wäßrigen Lösung der Schutzkolloide hergestellt. Geeignete Apparaturen sind beispielsweise Homogenisatoren, die nach dem Hochdruckentspannungsprinzip arbeiten, z.B. Lab 100 der Firma APV Gaulin.

Im allgemeinen verfährt man so, daß man das feste Diketen aufschmilzt, zu der auf 80 bis 85°C erwärmten wäßrigen Lösung eines Schutzkolloids zufügt und die Mischung unter Einwirkung von Scherkräften homogenisiert. Die Ester der Formel I können bei der Herstellung der Alkyldiketenemulsionen getrennt vom Alkyldiketen oder auch in Mischung mit dem geschmolzenen Alkyldiketen der wäßrigen, schutzkolloidhaltigen Lösung zugesetzt und homogenisiert werden. Nach dem Homogenisierschritt wird die erhaltene Emulsion auf Umgebungstemperatur abgekühlt. Der pH-Wert der Alkyldiketenemulsionen liegt in dem Bereich von 2,9 bis 3,1, vorzugsweise 3,0. Bei der Herstellung der Alkyldiketenemulsionen kann man gegebenenfalls noch weitere Hilfsstoffe, wie Ligninsulfonat, Formalin oder Propionsäure zusetzen.

Die konzentrierten Alkyldiketenemulsionen werden vor der Anwendung als Masse-Leimungsmittel für Papier durch Zugabe von Wasser auf Konzentrationen von 0,08 bis 0,5 Gew.-% an Alkyldiketen verdünnt.

Die in den Beispielen angegebenen Teile sind Gewichtsteile, die Angaben in Prozent bezieht sich jeweils auf das Gewicht der Stoffe.

### Beispiel 1

Man stellt zunächst eine 2 %ige wäßrige Suspension einer handelsüblichen kationischen Stärke her (Substitutionsgrad 0,02), in dem man die erforderliche Menge an kationischer Stärke in Wasser suspendiert und danach soviel Schwefelsäure zusetzt, daß der pH-Wert 3 beträgt. Danach erhitzt man die Stärkesuspension innerhalb von 1 Stunde auf eine Temperatur von 95°C, rührt das Reaktionsgemisch 1 Stunde bei dieser Temperatur und läßt die so erhaltene wäßrige Lösung dann abkühlen.

Zu 78 Teilen der obenbeschriebenen 2 %igen wäßrigen Stärkesuspension, die eine Temperatur von 85°C hat, gibt man eine auf 90°C erhitzte Schmelze aus 20 Teilen Stearyldiketen und 2 Teilen Stearinsäureoleylester zu und behandelt die Mischung 3 Minuten mit dem Turrax. Anschließend wird die Emulsion bei einer Temperatur von 70°C und einem Druck von 150 bar zweimal in einem Lab 100 homogenisiert und danach auf Raumtemperatur abgekühlt. Man erhielt eine stabile, 20 %ige wäßrige Stearyldiketenemulsion, die nach einer Lagerung von 30 Tagen bei 20-25°C noch immer stabil war. Ein Aufrahmen oder Festwerden der Emulsion wurde nicht beobachtet.

### Beispiel 2

Beispiel 1 wurde mit den Änderungen wiederholt, daß man zu 76 Teilen der Stärkesuspension 20 Teile Stearyldiketen und 4 Teile Ölsäurestearylester zugab. Man erhielt eine stabile, wäßrige 20 %ige Stearyldiketenemulsion, die nach einer Lagerung von 30 Tagen bei 20-25°C noch immer stabil war. Ein Aufrahmen oder Festwerden der Emulsion wurde nicht beobachtet.

### Vergleichsbeispiel

Das Beispiel 1 wurde mit der Ausnahme wiederholt, daß man das Emulgieren des Stearyldiketens in Abwesenheit von Stearinsäureoleylester bzw. Ölsäurestearylester durchführte. Man erhielt eine wäßrige Stearyldiketenemulsion, die nur 4 Tage bei 20-25°C lagerstabil war. Nach diesem Zeitraum wurde die Dispersion fest.

### Beispiel 3

Beispiel 1 wurde mit den Ausnahmen wiederholt, daß man zu 72 Teilen der Stärkesuspension eine Schmelze aus 20 Teilen Stearyldiketen und 8 Teilen Kohlensäureoleylstearylester zufügte. Man erhielt eine stabile, 20 %ige wäßrige Stearyldiketenemulsion, die nach einer 30-tägigen Lagerung bei 20-25°C noch immer stabil war.

### Beispiel 4

Beispiel 1 wurde mit den Ausnahmen wiederholt, daß man anstelle von Stearinsäureoleylester dieselbe Menge an Oleyl-N,N-distearylurethan verwendete. Man erhielt eine stabile, 20 %ige wäßrige Stearyldiketenemulsion, die nach 30-tägiger Lagerung bei 20-25°C noch immer stabil war.

### Beispiel 5

Beispiel 1 wurde mit den Ausnahmen wiederholt, daß man zu 66 Teilen der Stärkesuspension eine Schmelze aus 30 Teilen Stearyldiketen und 4 Teilen Kohlensäureoleylstearylester zufügte. Man erhielt eine stabile 30 %ige wäßrige Stearyldiketenemulsion, die nach einer Lagerung von 30 Tagen bei 20 bis 25°C noch immer stabil war.

### Beispiel 6

Beispiel 1 wurde mit den Ausnahmen wiederholt, daß man zu 76 Teilen der Stärkesuspension eine Schmelze aus 20 Teilen Stearyldiketen und 4 Teilen Ölsäureoleylester zufügte. Man erhielt eine stabile, 20 %ige wäßrige Stearyldiketendispersion, die nach einer 30-tägigen Lagerung bei 20-25°C noch immer stabil war.

### Beispiel 7

Beispiel 1 wurde mit den Ausnahmen wiederholt, daß man zu 64 Teilen der Stärkesuspension eine Schmelze aus 30 Teilen Stearyldiketen und 6 Teilen Ölsäureoleylester zufügte. Man erhielt eine stabile, 30 %ige wäßrige Stearyldiketendispersion, die nach einer 30-tägigen Lagerungen bei 20-25°C noch immer stabil war.

### Beispiel 8

Beispiel 1 wurde mit den Ausnahmen wiederholt, daß man zu 64 Teilen der Stärkesuspension eine Schmelze aus 30 Teilen Stearyldiketen und 6 Teilen Kohlensäuredioleylester zufügte. Man erhielt eine stabile, 30 %ige wäßrige Stearyldiketendispersion, die nach einer 30-tägigen Lagerung bei 20-25°C noch immer stabil war.

### Beispiel 9

Beispiel 1 wurde mit den Ausnahmen wiederholt, daß man zu 70 Teilen der Stärkesuspension eine Schmelze aus 20 Teilen Stearyldiketen und 10 Teilen Kohlensäuredioleylester zufügte. Man erhielt eine stabile, 20 %ige wäßrige Stearyldiketendispersion, die nach einer 30-tägigen Lagerung bei 20-25°C noch immer stabil war.

## Patentansprüche

1. Stabilisierte wäßrige C₁₄- bis C₂₂-Alkyldiketenemulsionen, dadurch gekennzeichnet, daß sie als wesentliche Bestandteile
(a) 10 bis 60 Gew.-% mindestens eines C₁₄- bis C₂₂-Alkyldiketens,
(b) 1 bis 10 Gew.-% mindestens eines Schutzkolloids und
(c) 0,1 bis 20 Gew.-% mindestens eines Esters der Formel 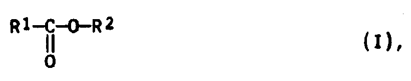
in der R¹ und R²
(1) C₁₄- bis C₂₂-Alkyl bedeuten, wobei sich R¹ und R² um mindestens 4 C-Atome in der Alkylkette unterscheiden,
(2) R¹ = C₁₄- bis C₂₂-Alkyl
R² = C₁₄- bis C₂₂-Alkenyl
(3) R¹ = C₁₄- bis C₂₂-Alkenyl
R² = C₁₄- bis C₂₂-Alkyl oder
(4) R¹ und R² gleiche oder verschiedene C₁₄- bis C₂₂-Alkenyl bedeuten,
eines Esters der Formel 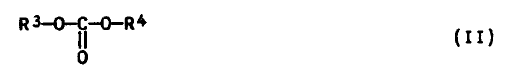 in der R³ und R⁴ gleiche oder verschiedene Alkyl- oder Alkenylreste mit 2 bis 22 C-Atomen bedeuten und R³ oder R⁴ mindestens 6 C-Atome aufweist, oder
eines Urethans der Formel 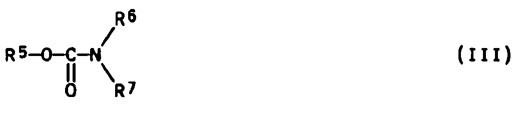 enthalten, in der R⁵, R⁶ und R⁷ gleiche oder verschiedene Alkyl- oder Alkenylreste mit 2 bis 22 C-Atome bedeuten und einer der Substituenten R⁵, R⁶ oder R⁷ mindestens 12 C-Atome aufweist,
und daß das Verhältnis der emulgierten Bestandteile (a):(c) 200:1 bis 1:1 beträgt.

2. Stabilisierte wäßrige C₁₄- bis C₂₂-Alkyldiketenemulsionen nach Anspruch 1, dadurch gekennzeichnet, daß sie
(a) 16 bis 40 Gew.-% mindestens eines C₁₄- bis C₂₂-Alkyldiketens,
(b) 1 bis 4 Gew.-% mindestens einer kationischen Stärke und
(c) 1 bis 10 Gew.-% mindestens eines Esters der Formeln I oder II oder ein Urethan der Formel III
enthalten.

3. Verwendung von Estern der Formel 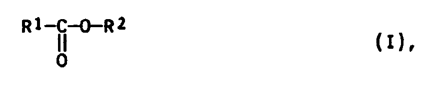 in der R¹ und R²
(1) C₁₄- bis C₂₂-Alkyl bedeuten, wobei sich R¹ und R² um mindestens 4 C-Atome in der Alkylkette unterscheiden,
(2) R¹ = C₁₄- bis C₂₂-Alkyl
R² = C₁₄- bis C₂₂-Alkenyl
(3) R¹ = C₁₄- bis C₂₂-Alkenyl
R² = C₁₄- bis C₂₂-Alkyl oder
(4) R¹ und R² gleiche oder verschiedene C₁₄- bis C₂₂-Alkenyl bedeuten,
oder von Estern der Formel 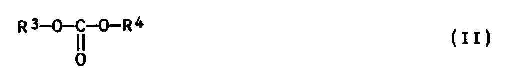 in der R³ und R⁴ gleiche oder verschiedene Alkyl- oder Alkenylreste mit 2 bis 22 C-Atomen bedeuten und R³ oder R⁴ mindestens 6 C-Atome aufweist, oder von Urethanen der Formel 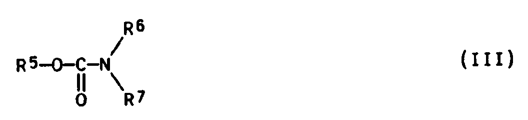 in der R⁵, R⁶ und R⁷ gleiche oder verschiedene Alkyl- oder Alkenylreste mit 2 bis 22 C-Atome bedeuten und einer der Substituenten R⁵, R⁶ oder R⁷ mindestens 12 C-Atome aufweist,
als Stabilisator zur Herstellung von wäßrigen C₁₄- bis C₂₂-Alkyldiketenemulsionen, die mindestens 10 Gew.-% Alkyldiketen emulgiert enthalten, in Mengen von 0,5 bis 50 Gew.-% bezogen auf Alkyldiketen.

4. Verwendung nach Anspruch 3, dadurch gekennzeichnet, daß man als Ester der Formel I
Stearinsäurebehenylester,
Behensäurestearylester,
Stearinsäureoleylester,
Ölsäurestearylester,
Ölsäurebehenylester,
Ölsäureoleylester
oder deren Mischungen einsetzt.

5. Verwendung nach Anspruch 3, dadurch gekennzeichnet, daß man als Ester der Formel II
Kohlensäureoleylstearylester,
Kohlensäurebehenyloleylester,
Kohlensäurebehenylstearylester
Kohlensäuredioleylester
oder deren Mischungen einsetzt.

6. Verwendung nach Anspruch 3, dadurch gekennzeichnet, daß man als Urethan der Verbindung III Oleyl-N,N-distearylurethan einsetzt.

7. Verwendung nach einem der Ansprüche 3 bis 6, dadurch gekennzeichnet, daß man die Verbindungen der Formeln I bis III in Mengen von 1 bis 10 Gew.-% bei der Herstellung von wäßrigen Alkyldiketenemulsionen einsetzt, die 16 bis 40 Gew.-% Alkyldiketen emulgiert enthalten.

## Claims

1. A stabilized aqueous C₁₄- to C₂₂-alkyldiketene emulsion, which contains as essential constituents
(a) from 10 to 60% by weight of at least one C₁₄- to C₂₂-alkyldiketene,
(b) from 1 to 10% by weight of at least one protective colloid and
(c) from 0.1 to 20% by weight of at least one ester of the formula 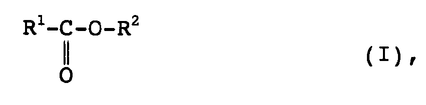
where
(1) R¹ and R² are each C₁₄- to C₂₂-alkyl, in which case R¹ and R² differ by at least 4 C atoms in the alkyl chain,
(2) R¹ = C₁₄- to C₂₂-alkyl
R² = C₁₄- to C₂₂-alkenyl
(3) R¹ = C₁₄- to C₂₂-alkenyl
R² = C₁₄- to C₂₂-alkyl or
(4) R¹ and R² are identical or different C₁₄- to C₂₂-alkenyl,
of an ester of the formula 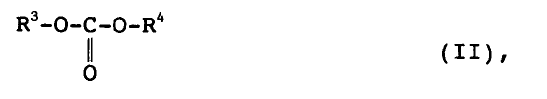 where R³ and R⁴ are identical or different alkyl or alkenyl having 2 to 22 C atoms and R³ or R⁴ has at least 6 C atoms, or
of a urethane of the formula 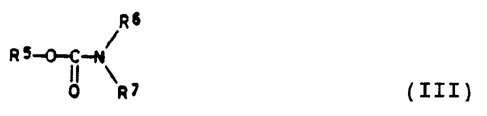 where R⁵, R⁶ and R⁷ are identical or different alkyl or alkenyl having 2 to 22 C atoms and one of the substituents R⁵, R⁶ or R⁷ has at least 12 C atoms,
and the ratio of the emulsified constituents (a):(c) is 200:1 to 1:1.

2. A stabilized aqueous C₁₄- to C₂₂-alkyldiketene emulsion as claimed in claim 1, which contains
(a) from 16 to 40 % by weight of at least one C₁₄- to C₂₂-alkyldiketene,
(b) from 1 to 4% by weight of at least one cationic starch and
(c) from 1 to 10% by weight of at least one ester of the formula I or II or a urethane of the formula III.

3. The use of esters of the formula 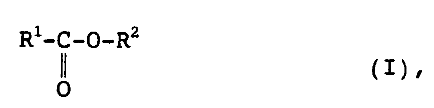 where
(1) R¹ and R² are each C₁₄- to C₂₂-alkyl, in which case R¹ and R² differ by at least 4 C atoms in the alkyl chain,
(2) R¹ = C₁₄- to C₂₂-alkyl
R² = C₁₄- to C₂₂-alkenyl
(3) R¹ = C₁₄- to C₂₂-alkenyl
R² = C₁₄- to C₂₂-alkyl or
(4) R¹ and R² are identical or different C₁₄- to C₂₂-alkenyl,
or of esters of the formula 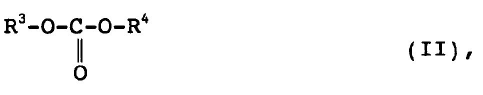 where R³ and R⁴ are identical or different alkyl or alkenyl having 2 to 22 C atoms and R³ or R⁴ has at least 6 C atoms, or
of urethanes of the formula 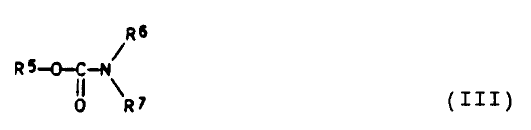 where R⁵, R⁶ and R⁷ are identical or different alkyl or alkenyl having 2 to 22 C atoms and one of the substituents R⁵, R⁶ or R⁷ has at least 12 C atoms,
as stabilizer for the preparation of aqueous C₁₄- to C₂₂-alkyldiketene emulsions, which contain at least 10% by weight of emulsified alkyldiketenes, in amounts of from 0.5 to 50% by weight, based on alkyldiketene.

4. The use as claimed in claim 3, wherein the esters of the formula I used are
behenyl stearate,
stearyl behenate,
oleyl stearate,
stearyl oleate,
behenyl oleate,
oleyl oleate
or mixtures thereof.

5. The use as claimed in claim 3, wherein the esters of the formula II used are
oleyl stearyl carbonate,
behenyl oleyl carbonate,
behenyl stearyl carbonate,
dioleyl carbonate
or mixtures thereof.

6. The use as claimed in claim 3, wherein the urethane of the compound III used is oleyl-N,N-distearyl-urethane.

7. The use as claimed in any of claims 3 to 6, wherein the compounds of the formulae I to III are used in amounts of from 1 to 10% by weight in the preparation of aqueous alkyldiketene emulsions which contain from 16 to 40% by weight of emulsified alkyldiketene.

## Revendications

1. Emulsions aqueuses et stabilisées d'alkyl(C₁₄-C₂₂)dicétènes, caractérisées en ce qu'elles contiennent, à titre de constituants essentiels,
(a) 10 à 60% en poids d'au moins un alkyl(C₁₄-C₂₂)dicétène,
(b) 1 à 10% en poids d'au moins un colloïde protecteur et
(c) 0,1 à 20% en poids d'au moins un ester de la formule 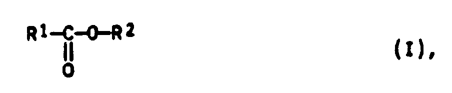
dans laquelle R¹ et R² représentent
(1) des radicaux alkyle en C₁₄ à C₂₂, où R¹ et R² diffèrent d'au moins 4 atomes de carbone dans la chaîne alkyle,
(2) R¹ = alkyle en C₁₄ à C₂₂
R² = alcényle en C₁₄ à C₂₂,
(3) R¹ = alcényle en C₁₄ à C₂₂
R² = alkyle en C₁₄ à C₂₂, ou bien
(4) R¹ et R² représentent des radicaux alcényle en C₁₄ à C₂₂, identiques ou différents,
d'un ester de la formule 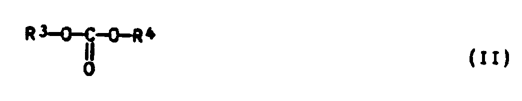 dans laquelle R³ et R⁴ représentent des radicaux alcényle ou alkyle, identiques ou différents, comportant de 2 à 22 atomes de carbone et R³ ou R⁴ comporte au moins 6 atomes de carbone, ou bien
d'un uréthanne de la formule 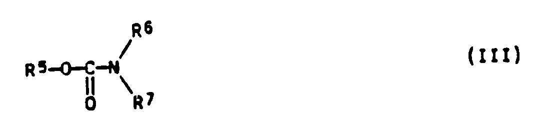 dans laquelle R⁵, R⁶ et R⁷ représentent des radicaux alcényle ou alkyle, identiques ou différents, comportant de 2 à 22 atomes de carbone et l'un des substituants R⁵, R⁶ et R⁷ comporte au moins 12 atomes de carbone,
et en ce que le rapport des constituants émulsionnés (a):(c) varie de 200:1 à 1:1.

2. Emulsions aqueuses et stabilisées d'alkyl(C₁₄-C₂₂)dicétènes selon la revendication 1, caractérisées en ce qu'elles contiennent
(a) 16 à 40% en poids d'au moins un alkyl(C₁₄-C₂₂)dicétène,
(b) 1 à 4% en poids d'au moins un amidon cationique et
(c) 1 à 10% en poids d'au moins un ester de la formule I ou de la formule II, ou d'un uréthanne de la formule III.

3. Utilisation d'esters de la formule 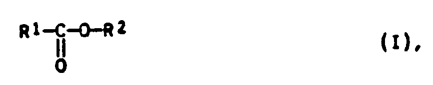 dans laquelle R¹ et R² représentent
(1) des radicaux alkyle en C₁₄ à C₂₂, où R¹ et R² diffèrent d'au moins 4 atomes de carbone dans la chaîne alkyle,
(2) R¹ = alkyle en C₁₄ à C₂₂
R² = alcényle en C₁₄ à C₂₂,
(3) R¹ = alcényle en C₁₄ à C₂₂
R² = alkyle en C₁₄ à C₂₂, ou bien
(4) R¹ et R² représentent des radicaux alcényle en C₁₄ à C₂₂, identiques ou différents,
d'un ester de la formule 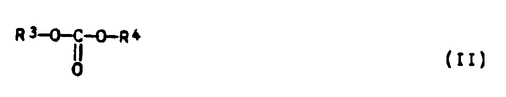 dans laquelle R³ et R⁴ représentent des radicaux alcényle ou alkyle, identiques ou différents, comportant de 2 à 22 atomes de carbone et R³ ou R⁴ comporte au moins 6 atomes de carbone, ou bien
d'un uréthanne de la formule 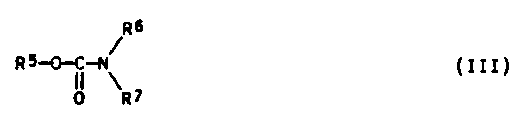 dans laquelle R⁵, R⁶ et R⁷ représentent des radicaux alcényle ou alkyle, identiques ou différents, comportant de 2 à 22 atomes de carbone et l'un des substituants R⁵, R⁶ et R⁷ comporte au moins 12 atomes de carbone,
à titre d'agents de stabilisation pour la préparation d'émulsions aqueuses d'alkyl(C₁₄-C₂₂)dicétènes, qui contiennent au moins 10% en poids d'alkyldicétène en émulsion, en proportions de 0,5 à 50% en poids, par rapport à l'alkyldicétène.

4. Utilisation suivant la revendication 3, caractérisée en ce que l'on utilise, à titre d'ester de la formule I,
le stéarate de béhényle,
le béhénate de stéaryle,
le stéarate d'oléyle,
l'oléate de stéaryle,
l'oléate de béhényle,
l'oléate d'oléyle,
ou leurs mélanges.

5. Utilisation suivant la revendication 3, caractérisée en ce que l'on utilise, à titre d'esters de la formule II,
le carbonate d'oléyle et de stéaryle,
le carbonate de béhényle et d'oléyle,
le carbonate de béhényle et de stéaryle,
le carbonate de dioléyle,
ou leurs mélanges.

6. Utilisation suivant la revendication 3, caractérisée en ce que l'on utilise l'oléyl-N,N-distéaryluréthanne à titre d'uréthanne de la formule III.

7. Utilisation suivant l'une quelconque des revendications 3 à 6, caractérisée en ce que l'on utilise les composés des formules I à III en proportions de 1 à 10% en poids au cours de la préparation d'émulsions aqueuses d'alkyldicétènes qui contiennent de 16 à 40% en poids d'alkyldicétène en émulsion.
